# EUROPEAN PATENT APPLICATION

(11) **EP 1 479 779 A1**
(43) Date of publication of application: **24.11.2004**
(21) Application number: 03447112.8
(22) Date of filing: 19.05.2003
(51) Int. Cl.: C12Q 1/68

(54) **Biological signature of manufactured products**

(71) Applicant: Eppendorf Array Technologies SA, B-5000 Namur (BE)
(72) Inventor: Remacle, José, 5020 Malonne (BE)
(74) Representative: Van Malderen, Joelle

(57) **Abstract**

The present invention is related to a method of identification of a non-biological manufactured product by a labelling (indiyidualisation) of said product with a specific nucleotide sequence bound to the external surface of said product or incorporated inside the elements of said product and followed by its specific detection through an hybridisation of the specific nucleotide sequence upon a corresponding nucleotide sequence, said hybridisation resulting in a detectable signal, and possibly a detection or quantification of said signal and a discrimination of said manufactured product from other products.

## Description

### Field of the invention

The present invention is related to a method for obtaining the individualisation by a biological signature of non-biological manufactured products by a specific biological labelling and thereafter, the detection and the identification of said manufactured product.

The present invention is also related to said manufactured product comprising said biological labelling.

### State of the art

The labelling and/or identification of a manufactured product has been developed by using the so-called barcode which is a technique for describing character (alphabeticals, numerals, symbols, etc.) by a combination of parallel (vertical) lines, hereafter referred to as bars of different thickness (width) or arranged at different intervals. Each barcode (symbol) will present binary information which can be optically read by optical reading means such as an optical scanner.

These barcodes are printed on labels and alike which are attached to books, food products and alike articles or directly printed on the surface of articles (surface of wrappings for articles), thereby permitting them to be imputed by input means (reading means) which quickly input binary information at libraries. The two values (0 and 1) of the binary information are distinguished from each other by differences in reflectance of darker and lighter portions of the barcode, darker portions having a lower reflectance (referred to as black bars) and recognised (read) as one (the key 1 of the binary value), while the light portions having a higher reflectance are recognised (read) as the other (i.e. 0). Recently, it has also-been decided to integrate colour in a barcode system.

However, this device still represents the drawbacks of being highly visible upon the manufactured product and should be either printed or presented upon a label and put upon the surface of the manufactured product.

### Aims of the invention

The present invention aims to provide a new method and means for improving labelling of manufactured products.

A particular aim of the present invention is to provide such method and means which are not directly recognised by the consumer and do not necessarily form the incorporation of a labelling strip upon the surface of the product, but are integrated directly upon the surface of any kind of product or inside the product and made visible or detectable by an appropriate treatment of the surface.

A further aim of the present invention is to provide inside or upon the surface of a manufactured product a large number of individual biological signatures, which could be present as recorded biological information units. The method also provides a large number of individual signatures

A last aim of the present invention is to provide a method for the storage of information by using said biological signature upon manufactured products as recorded biological information units.

### Summary of the invention

The invention concerns a method for obtaining the individualisation or biological signature of a "non-biological" manufactured product by a labelling of said product with a specific nucleotide sequence.

The present invention is also related to a method appropriate for the specific revelation of said nucleotide sequence, preferably through a hybridisation with a complementary nucleotide sequence and the detection of a signal resulting from said hybridisation.

A biological signature means the incorporation of a specific biological molecule, in particular a nucleotide sequence present upon the surface or inside the elements (fibres, metallic or polymeric surfaces,...) of the product to be recognised. The specificity of biological labelling or biological signature is obtained by using a specific and previously determined (synthetic) nucleotide sequence which in appropriate conditions hybridise to a complementary nucleotide sequence resulting in a signal which is read by appropriate means.

A "non-biological" manufactured product means any consumable solid product or packaging of a consumable solid liquid or gaseous product, which can be made or used in any kind of industry, including agriculture, but which is not made of biological elements comprising nucleotide acids (such as of animals and plants, or parts (or portions such as meat, skin, seeds, flowers, ...) of said animals or plants). However, the manufactured product can be the packaging of said "biological materials".

Furthermore, said "non biological" manufactured product or its packaging preferably do not comprise or produce denaturing elements (such as enzymes, organic acids,...) which may destroy the biological signature. The manufactured product is also different from known solid supports which are already used for biological detection, such as micro-well plates, biochips, etc.

The specific nucleotide sequence incorporated in or present upon the surface of said manufactured products gives, by its specific detection (identification and possibly, quantification) a specific biological signature and allow a detection and therefore the traceability of said manufactured products.

According to the invention, said nucleotide sequence is bound to or is present in the said products and fixed to the molecules of said products through a common link in order to be accessible to its complementary nucleotide sequence in order to result in a signal to be detected (reading allowing an identification and possibly a quantification of said nucleotide sequence).

Advantageously, said nucleotide sequence is bound to a specific material called hereafter nucleotide bearing material (or NBM), which is previously incorporated or bound to the molecule of said manufactured products so as to be thereafter detected. Such NBM is added during the manufacturing step of the product and is preferably present on the whole external surface of the product or only in special portions of the product or its surface.

The NBM is preferably incorporated in elements used in the polymerisation of the manufactured products or in the packaging of said manufactured products.

Advantageously, the NBM is composed of microbeads, preferably having a size comprised between 0.001 and about 1000 µm, more preferably between about 0.1 and 10 µm, upon which one or more nucleotide sequences can be covalently bound.

Said NBM, including the microbeads, is preferably made of the same organic (polymeric matrix) or inorganic (metallic or magnetic) compounds than the manufactured products to be recognised.

The specific nucleotide sequence bound to the NBM or directly incorporated upon the surface or in the manufactured product to be recognised, presents a sequence of between about 10 and about 1000 bases, preferably between 20 and about 200 bases length.

Furthermore, the nucleotide sequence is preferably bound upon the manufactured products or upon the NBM through a linker (spacer), preferably through a nonspecific nucleotide sequence of more than 10 bases, preferably more than 50 bases, or more than 100 bases but preferably lower than 1000 bases, 500 bases or 200 bases. Such linker or spacer can be also a polymeric chain of at least 20 atoms, preferably 50 atoms or more preferably 100 atoms but lower than 1000 atoms, 500 atoms or 200 atoms. Preferably, the polymeric chain is a polyethylene glycol (PEG) chain.

In one embodiment of the invention, the NBM also comprises branch polymer upon its surface allowing a preferred binding of said nucleotide sequence. The detection (identification or quantification) of the nucleotide sequence bound upon or present inside the manufactured product is preferably realised by the detection of a signal resulting from the hybridisation of said specific nucleotide sequence to its specific corresponding nucleotide sequence.

In a specific feature of the invention, the nucleotide sequence is not detectable by itself, but is only detectable after hybridisation with its specific corresponding nucleotide sequence. In still a preferred embodiment, signal is obtained through further specific processing of the surface to obtain a detectable signal. Said signal of detection is preferably measured by colorimetry, spectrometry, fluorescence, chemoluminescence, electro-luminescence, electric or other analysis methods applied to genetic sequence detection, which can be thereafter detected and/ or recorded by a known detection means.

One or two of said sequences (specific integrated nucleotide sequences upon or inside the manufactured products or its corresponding nucleotide sequence) is advantageously labelled with a first member of a binding pair such as biotin, which then react with the second member of said specific binding pair (streptavidin) bound to gold micro particles or anti-biotin antibodies labelled with gold nano particles.

Such gold particles are either detected as such, as a catalyst for a possible silver precipitation. Said silver deposit upon the surface of the manufactured products or inside the elements of the manufactured products results in a darkening of the surface, which is easily detected by various means. Said silver deposit is made of particles having scattering or a defraction power of a light beam. One particularity of silver is its heat and electric properties which are detectable when different from the surface of the manufactured product on which is bound the signature. Of particular interest is the heat exchange or conductivity of the silver and its electric high conductivity or capacitance or low resistance.

Said signal may also result from an enzymatic reaction such as the one obtained with the use of peroxydase or alkaline phosphatase advantageously coupled to streptavidin. The combination of these two systems of detection is proposed in order to obtain firstly, a colorimetric detection and thereafter, a detection through a metallic precipitate.

In a particular embodiment, the complementary nucleotide sequence is labelled with fluorochrome molecules such as Cy3 , Cy5 or Cy7 and fluorescence is detected on the surface of the manufactured product.

Advantageously, the preferred specific nucleotide sequence to be recognised by its complementary nucleotide sequence, comprises nucleotides of between about 5 and about 100 bases, more preferably about 15 and about 40 bases.

The biological signature obtained by the method according to the invention has the advantage of being invisible by human eyes or light detection upon the product, but is specifically detected and/or quantified thereafter. If necessary, said metallic or colorimetric precipitate provides a specific design, including barcodes (system of lines with the same or different widths which are read by a barcode reader).

Typical applications of the invention is to provide biological signatures to unique or expensive materials and goods. Particularly indicated are joellery, cars or parts of the cars, art pieces, booknotes. The biological signature is particularly useful tool to provide identification of identity cards, passports or trade name materials. It is also easily applied when incorporated into plastic film especially transparent plastic where the detection is particularly easy to obtain. It is also useful to determine the origin of the production or to follow the traceability of the product. Individual signature of any personal manufactured products is achieved by using a sequence of nucleotides being specific of each person; the sequence can be one part of the personal genomic DNA sequence.

In one particular embodiment, the non-biological manufactured product according to the invention contains one or more different or similar nucleotide sequences, which comprise (recorded) biological information units made of the base of said nucleotide sequence(s), which is therefore used for storage of information. Said (recorded) biological information units is present on the same or upon different nucleotide sequence(s), the special disposition of said biological information units being part of an information storage system. Therefore, another aspect of the present invention is related to a method for the storage of information by using the biological information units present upon said "identical or different" nucleotide sequence put upon the surface of a manufactured product or inside said manufactured product.

### Description of a preferred embodiment of the invention

Nucleotides sequences are continuous chains of nucleotides formed from 4 different bases.

The number of specific sequences is directly dependent of its length being 4ⁿ with n the number of nucleotides of the sequences. A typical 20 bases long sequence gives a number of different combinations so high making it unique in most circumstances.

Another feature of a nucleotide chain is its possible recognition by a complementary sequence chain. Hybridisation of the complementary chain is the result of the recognition between G=C and A=T.

In this invention, a large number of possible sequences is used and their specific recognition by complementary sequence is obtained in order to label products especially manufactured products in a very specific and even individual way.

The invention is especially useful for labelling produced product and to be able to identify them even after their incorporation as pieces in another elaborated product.

A nucleotide sequence of at least 5 bases is bound to solid substrate being for example glass or silica beads in order to obtain a nucleotide bearing material (NBM).

Biotin molecules are attached to the oligonucleotide used for the specific hybridisation with the nucleotide sequence present on the NBM. Biotin is then recognised with streptadin-monogold particles or anti-biotin monogold particles and detected as such if sufficient particles are present on the surface. A precipitation of Ag particles is preferably performed in order to amplify the particles size until about 0,8 mum. size. The detection of these particles is best observed by using a light scattering or diffraction detection.

A typical detector used is a laser beam illuminating the surface of the product and a photodiode located laterally to detect the diffracted light. The detection is obtained with transparent material such as polycarbonate or polyethylene, polyacryate, polymethyl, metacrylate or with opaque or dark material with low diffraction yield.

The inventors have also discovered that the nucleotide sequence present on the NBM is much better detected, when present at the end of a linker (spacer) fixed upon the support (but which should be not too long). The detection of the biological signature is better when the said specific sequence is located at one end of a 5 to 500 nucleotide spacer sequence. In one particular embodiment, a polymer linker being either a monochain or a ramified polymer (like a branched polymer), is being grafted with the specific nucleotide. A long single polynucleotide strand chain is considered as containing a linker if only one part of the sequence is used for the detection with a complementary labelled probe. A typical chain length of between 50 to 500 bases is easily produced by PCR amplification and can be specifically detected with a probe of between 10 to 30 bases. Long linkers or spacers have the drawbacks of being possibly cut by overbreaking during the product manufacturing thus decreasing the detection yield. Breaks are due to shearing forces, overheating and particular wavelength radiations. Example 1 provides an example of NBM being glass beads activated in order to fix capture probes. Similarly, polystyrene beads are activated by oxidation by KMnO₄ and the carboxylated functions will bind the amino-probes in the presence of coupling agents such as carbodiimide.

Rather than being fixed on a solid material like beads, the nucleotides are fixed directly on the polymers chains or on the monomers of said plastic elements and incorporated to as such during the manufacturing of the product or in some of its part. Monomers or polymers with isothiocyanate, acrylate, epoxyde, aldehyde or other reactive groups are substituted with aminated nucleotides (obtained by chemical synthesis or after amplification by PCR by using one amino ended primer). The nucleotide substituted monomers or partially polymerised or polymers are then added in the manufacturing process of one part at least of the manufactured product. Such manufacturing process include moulding or extracting step. In order to optimise the use of NBM, it is preferred to add the NBM (with the nucleotide sequence or not) in the mould or in the extruder in such a way as to obtain the NBM on the surface of the product so as to be detectable there after.

Another preferred method is to use the nucleotide bearing polymer as an external coating of the surface of the manufactured product. Monomers are also usable by being applied on the product surface by process like spin coating and then polymerisation. One preferred process is coating followed by UV irradiation to initiate the polymerisation process. A lot of different monomers, especially having vinyl groups are susceptible to polymerize either as linear or three dimensional molecules after initialisation by UV light.

In one particular application, the deposition of the polymers at the surface of the product is spatially specific making the spatial arrangement of the nucleotide polymer typical of the product as explained here above for the NBM. One of such typical arrangement is lined of various size which after labelling are read by a code bar reader. The invention is not limited to a specific spatial arrangement since other geometrical forms such as squares, dots, rectangles or other can give specific features. A favourite application is the deposit of the NBM beads through an inject or piezo base delivery system automate in order to provide the spatially arrangement of the signal on the surface of a product.

In a particular embodiment, different NBM are used as means (biological information units) for obtaining an information storage. Nucleotide sequences contains part of the information in the sequence of the AGCT nucleotide. The number of combinations is n⁴, n being the number of nucleotides in the sequences. When two or more NBMs are present in the surface of a manufactured product and more particularly on the surface of a packaging, the number of combinations of the NBMs on a given surface is x^{y} with x the number of NBM at one position and y the number of NBMs positions on the surface. Since each NBM represent n⁴ combination of nucleotide sequences, the total number of combination for y NBMs will be n^{4y}. Combination numbers will be very high for nucleotide sequence of 10 (n=10) and with 10 NBM (y=10) on the surface, the possible combination will be 10⁴⁰.

For sequence of 15 nucleotides which is typical the sequence for which a specific hybridisation conditions can be worked out and for 10 NBM, the number of possible combinations will be 10⁶⁰ or almost unlimited.

Therefore, said NBMs are particularly efficient to store information. One example is a NBM containing a sequence of 15 nucleotides which are a 5 times repeat of 3 nucleotides. 64 of such NBM are constituted and used for information writing and storage. The NBM are then used as 64 bits and organised on the surface as bytes for storage, in the same way as bytes are used on computer or CD-ROM. The 5 times repeat is given as an example, the number of repeats being between 1 and 20, preferably between 3 and 10 and the number of different nucleotides is 2, 3, 4..

In another embodiment the NBM contain a sequence of 16 nucleotides which are a 4 times repeat of 4 nucleotides. 256 of such NBM are constructed and used for information writing and storage. The NBM beads are delivered on the surface of the packaging as individual leads or as row of beads. The reading is done by detection of the beads labelled with specific label probes or after hybridisation with specific label probes or after hybridisation with specific labelled complementary nucleotide sequences.

In one particular application, the NBM contain spaces for increasing availability of reaction with their corresponding specific probes as explained here above. According to the invention, the person skilled in the art may also use nucleotide sequences as a source of writing, storing and reading information which are now written, stored and read as binary information. The use of 4, 64 or even 256 or even higher number of information units (BIU) makes this storage of information particularly efficient (BIU= Biological Information Units). As explained here above the BIU will store any amount of information either as single NBM or as combination of several NBMs.

### Example

### 1.Binding of capture nucleotide sequences on silica beads

### Glass activation

Silica beads bearing silanol functions were first grafted with olefinic silane coupling agent in order to cover the surface with olefinic groups. Two grams of silica sample was immersed for 1h in a 3% anhydrous toluene solution of 7-octenyltrichlorosilane (ABCR (Germany, Karlsruhe). The samples were then cleaned three times by dipping in fresh toluene under mild agitation and three times in acetone to remove the excess physisorbed molecules, then dried in oven at 70 °C during 45 min.

### Olefinic oxidation.

The olefinic functions present either on glass or polymers were oxidized in the following way. Silica beads were dipped into a solution of 0.01M Phosphate buffer at pH 7.4 containing 0.5 M NaIO4 and 20 mM KMnO4 under mild agitation during 1h, washed six times with water, three times with acetone and dried at 20°C. Sample was stored at 4°C.

### Grafting of aminated DNA nucleotide sequences on silica beads

### Capture nucleotide sequence synthesis

Capture nucleotide sequences are synthesized by PCR using primers and method described by Zammatteo *et al.*(1997). *Anal. Biochem.* **253**, 180-189). One primer Mie4 bears an amine group at its 5' terminus and the length of amplicons is 255 bp. The sequences of the primers were MiE4 : ccaagcggcctctgataaccaag and MiE6 : gtacaggggactctgggggtgac....

The amplified sequence was a part of the major immediate early gene of cytomegalovirus genome (HCMV strain AD169). During the PCR process, biotinylated nucleotides are incorporated to generate 255 base long biotinylated amplicons bearing an aminated end. Amplified DNA is separated from unincorporated nucleotides and primers by chromatography on High Pure PCR Product Purification Kit. DNA concentration is then estimated on agarose gel electrophoresis.

### Binding of single stranded capture nucleotide sequences to aldehyde sample

500 mgr of silica beads are diluted to a concentration of 100 nM in SSC 3X buffer pH 5, 0.05% SDS. Grafting is performed during 1 h under mild agitation. After incubation, sample is washed once with 0.2% SDS, twice with water, then incubated for 5 min with sodium borohydride solution [2.5 mg/ml dissolved in a PBS/Ethanol solution (75/25)], once with water and finally 3 min in boiling water to obtain single stranded nucleotide sequences on the surface. Beads are resuspended in 50 ml of water.

### Test of DNA binding on beads.

To confirm the presence of biotinylated DNA on the surface of beads (possibly integrated in the packaging of a manufactured product such as a cigarette box), the detection of biotin on beads with DNA bound and on negative control beads without DNA. 2µl of silice beads are washed 4X2min with a 10 mM maleate buffer containing 15mM NaCl and 0.1% tween pH 7.5 (Washing buffer). The beads are then incubated for 45 min with streptavidin-HRP conjugate diluted 1000X in a blocking solution. The beads are then washed 5X2min in the same washing buffer then incubated 10 min in TMB solution to detect a blue colour appearing were HRP is present. The solution becomes yellow and 100µl of this solution is quantified by a colorimeter at 450 nm. An optical density of 3.5 for beads with DNA bound and an optical density of 0.2 for negative control beads can be obtained upon the surface of the packaging.

### 2. Identification of the presence of a DNA sequence on the beads

Detection for the presence of the particular DNA sequence on the beads is performed in the same way as in example 1 but with the beads bearing non-biotinylated DNA strand. The detection is then performed with DNA binding performed using the complementary sequence labelled with biotin as described by Zammatteo et al. (1997) ( *Anal. Biochem.* **253,** 180-189).

For the detection of the DNA sequence on a surface of a manufactured product, the DNA-bearing beads were deposit inside a polypropylene sheet and on a cellulose surface. The polymers surfaces are sticked to the surface of the manufactured product or its packaging.

The presence of the DNA on the surface of the beads is detected in colorimetry following this protocol : Slides are washed 4X2min with a 10 mM maleate buffer containing 15mM NaCl -pH 7.5 (Washing buffer). The slides are then incubated for 45 min with streptavidin-gold nanoparticle conjugate diluted 100X in a 100mM maleate buffer containing 150mM NaCl and 0.01% milk powder. The slides are then washed 5X2min in the same washing buffer.

The slides are incubated at room temperature for 10 min in 800µl of Silver Blue solution in the combination of 1/1 volume of Silver Blue A and Silver Blue B (EAT, Namur, Belgium). The silver blue allows the precipitation of silver catalyzed by the presence of the gold nanoparticles present on the DNA to be detected

The reaction of Silver precipitation is stopped by washing the slides in water for 2 min.

## Claims

1. A method of identification of a non-biological manufactured product by a labelling (individualisation) of said product with a specific nucleotide sequence bound to the external surface of said product or incorporated inside the elements of said product and followed by its specific detection through an hybridisation of the specific nucleotide sequence upon a corresponding nucleotide sequence, said hybridisation resulting in a detectable signal, and possibly a detection or quantification of said signal and a discrimination of said manufactured product from other labelled or non labelled products.

2. A method according to claim 1, wherein the specific nucleotide sequence is bound by a covalent link to the surface of a nucleotide bearing material (NBM) incorporated and added upon the surface of the manufactured product or incorporated inside the elements of the product.

3. The method according to claim 2, wherein said nucleotide bearing material is made of microbeads having a size comprised between about 0.01 and 1000 µm, more preferably between about 0.1 and about 10 µm.

4. The method according to any of the preceding claims, wherein the specific nucleotide sequence is bound to the nucleotide bearing material surface of upon the surface of the product or incorporated inside the elements of the product being at least 20 atoms, more preferably at least 50 atoms, more preferably 100 atoms, and being attached through a linker (or spacer) of more than 10 bases, preferably more than 50 bases, more specifically more than 100 bases.

5. The method according to any of the preceding claims, wherein the nucleotide sequence presents a length comprised between about 10 and about 1000 bases, more preferably between 20 and about 200 bases.

6. The method according to any of the preceding claims, wherein the specific nucleotide sequence contains a detectable probe and wherein the surface of the product is treated with a specific reagent in order to obtain at the localisation of the bound nucleotide sequence a colorimetric detectable signal.

7. The method according to any of the preceding claims, wherein the specific nucleotide sequence contains a detectable probe and wherein the surface of the product is treated with a specific reagent in order to obtain at the localisation of the bound nucleotide sequence a fluorescent detectable signal

8. A non-biological manufactured product having a specific nucleotide sequence bound to the external surface of a manufactured product or incorporated inside the elements of a manufactured product allowing its specific detection through an hybridisation of said specific nucleotide sequence upon a corresponding nucleotide sequence resulting in detectable signal.

9. A non-biological manufactured product according to claim 8 wherein Biological Information Units are present in the nucleotide sequence and are used for the storage of information.

10. A non-biological manufactured product according to claim 9 wherein several Biological Information Units are present upon the surface of the product having the same or different nucleotide sequences and wherein the spacial disposition of said Biological Information Units is part of the information storage.

11. A method for the storage information wherein a non-biological manufactured product is labelled by the method according to any of the preceding claims 1 to 7 and wherein said nucleotide sequence comprises one or more Biological Information Units used for the storage of information.

12. The method according to the claim 11 wherein said Biological Information Units are present according to a spatial disposition which are part of an information storage.
